# FASCICULE DE BREVET EUROPEEN

(11) **EP 4 304 685 B1**
(45) Date de publication et mention de la délivrance du brevet: **18.12.2024**
(21) Numéro de dépôt: 22714476.3
(22) Date de dépôt: 07.03.2022
(51) Int. Cl.: A61M 15/08, A61M 11/00, A61M 15/00, B05B 11/02

(54) **DISPOSITIF DE DISTRIBUTION NASALE DE PRODUIT FLUIDE**
VORRICHTUNG ZUR NASALEN VERABREICHUNG EINES FLÜSSIGEN PRODUKTS
DEVICE FOR NASAL DELIVERY OF A FLUID PRODUCT

(30) Priorité: 09.03.2021 FR 2102304
(43) Date de publication de la demande: 17.01.2024
(73) Titulaire: APTAR France SAS, 27110 Le Neubourg (FR)
(72) Inventeur: HERRY, Serge, 76520 Franqueville Saint Pierre (FR)
(74) Mandataire: CAPRI
(86) Numéro de dépôt international: PCT/FR2022/050400
(87) Numéro de publication internationale: WO 2022/189742

(56) Documents cités:
- EP-A1- 0 308 524
- WO-A1-2014/147329
- WO-A2-2012/119153
- FR-A1- 3 120 534
- US-A1- 2012 085 345
- US-A1- 2014 060 535
- US-A1- 2019 282 497
- US-B1- 6 647 980

## Description

La présente invention concerne un dispositif de distribution nasale de produit fluide.

Un inconvénient des dispositifs de distribution nasale de produit fluide existants concerne leur compatibilité avec une utilisation pédiatrique. En effet, les dispositifs standards peuvent générer des sprays qui présentent une force d'impaction dans la narine trop importante pour des nouveau-nés ou des enfants en bas âge. Typiquement, un dispositif standard génère une force d'impaction d'environ 0,05 N à 0,1 N, ce qui est trop élevé pour une utilisation pédiatrique.

On connaît des dispositifs de distribution nasale de produit fluide divulgués dans les documents US 2012/085345 A1, US 6 647 980 B1, US 2019/282497 A1 et WO 2014/147329 A1.

La présente invention a pour but de fournir un dispositif de distribution nasale de produit fluide qui ne reproduit pas les inconvénients susmentionnés.

Plus particulièrement, la présente invention a pour but de fournir un tel dispositif de distribution nasale qui évite ou limite les risques lors d'une utilisation pédiatrique.

La présente invention a également pour but de fournir un tel dispositif de distribution nasale qui soit simple et peu coûteux à fabriquer et à assembler.

La présente invention a donc pour objet un dispositif de distribution nasale de produit fluide selon l'une des revendications indépendantes 1 et 2. Les revendications dépendantes définissent des modes de réalisation de l'invention.

Ces caractéristiques et avantages et d'autres de la présente invention apparaitront plus clairement au cours de la description détaillée suivante, faite en référence aux dessins joints, donnés à titre d'exemples non limitatifs, et sur lesquels :
La figure 1 est une vue schématique en section transversale d'un dispositif de distribution de produit fluide selon un mode de réalisation avantageux,
La figure 2 est une vue de détail en section transversale de la tête de distribution de la figure 1, et
La figure 3 est une vue schématique agrandie de l'orifice de distribution de la tête de la figure 2.

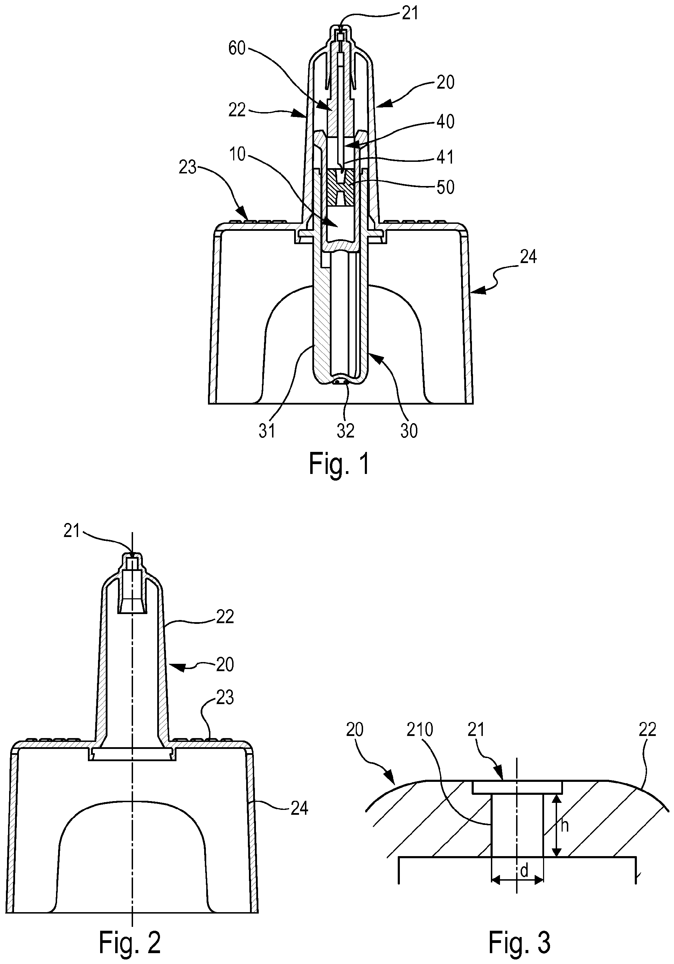

Les termes "proximal" et "distal" se réfèrent à l'orifice de distribution. Les termes "axial" et "radial" se réfèrent à l'axe central longitudinal du dispositif. Les termes "amont" et "aval" se réfèrent à la direction d'écoulement du fluide lors de l'actionnement. Les termes "haut" et "bas" se réfèrent à la position droite du dispositif représentée sur la figure 1.

Dans le mode de réalisation avantageux de la figure 1, un réservoir 10 contenant du produit fluide à distribuer, typiquement un liquide, est disposé à l'intérieur d'un corps formant une tête de distribution 20. Cette tête de distribution 20 comporte un orifice de distribution 21 orienté axialement. Cet orifice de distribution 21 sert à distribuer une dose de produit fluide hors de ladite tête de distribution 20 lors de l'actionnement du dispositif par un utilisateur.

La tête de distribution 20 comporte un embout nasal allongé 22 comportant à son extrémité axiale proximale ledit orifice de distribution 21, ainsi qu'un corps latéral 24, relié audit embout nasal 22 par une bride radiale 23.

Dans l'exemple de la figure 1, un insert creux 60 est disposé dans la tête de distribution 20, en amont dudit orifice de distribution 21, ledit insert creux 60 définissant un canal d'expulsion et, en coopération avec la paroi de fond de la tête de distribution 20, un profil de pulvérisation directement en amont dudit orifice de distribution 21.

Une canule creuse 40 est insérée et fixée dans ledit canal d'expulsion 61, ladite canule 40 comportant à son extrémité distale une pointe de perçage 41. Avantageusement, la canule 40 est emmanchée à force dans le canal d'expulsion.

Le réservoir 10 est dans l'exemple de la figure 1 formé par un corps creux et borgne, comportant une seule ouverture fermée par un bouchon-piston 50 et contenant une seule dose de produit fluide à distribuer un actionnement unique du dispositif.

Lors de l'actionnement, le réservoir 10 est déplacé axialement vers le haut par rapport à la tête de distribution 20 et donc par rapport à la canule 40. Le bouchon-piston 50 est ainsi poussé contre la pointe 41 de la canule 40 pour être percé, ce qui permet l'expulsion de tout ou partie du produit fluide contenu dans le réservoir 10.

Il est à noter que la présente invention peut aussi s'adapter à des dispositifs du type bidose ou multidoses, dans lequel le réservoir contient au moins deux doses de produit fluide à distribuer en au moins deux actionnements successifs du dispositif. Le document WO2014147329 décrit un exemple de dispositif bidose.

Des moyens d'actionnement 30 sont prévus pour permettre l'actionnement du dispositif de la figure 1.

Ces moyens d'actionnement 30 comportent un corps d'actionnement 31 mobile par rapport à la tête de distribution 20, ledit corps d'actionnement 31 coopérant avec ledit réservoir 10 pour le déplacer axialement par rapport à la tête de distribution 20, en direction de l'orifice de distribution 21.

Le fonctionnement du dispositif va être détaillé ci-après.

De manière classique, l'utilisateur place deux doigts sur la bride radiale 23 formée sur la tête de distribution 20, et appuie avec le pouce sur le fond axial distal 32 dudit corps d'actionnement 31. Lors d'un tel actionnement, le réservoir 10 est donc poussé axialement en direction de l'orifice de distribution 21, de sorte que la canule 40 perce le bouchon-piston 50. Le contenu du réservoir 10 est alors relié à l'orifice de distribution 21 et l'appui de l'utilisateur sur le corps d'actionnement 31 déplace le bouchon-piston 50 dans le réservoir 10 pour assurer la distribution du produit fluide.

L'orifice de distribution 21 comporte un cylindre axial 210 ayant une hauteur axiale h et un diamètre radial d.

Selon l'invention, ladite hauteur axiale h est égale à 0,6 mm et ledit diamètre radial d est égal à 0,4 mm où 0,5 mm.

Avec une telle géométrie de l'orifice de distribution 21, il est possible de réduire la force d'impaction du spray sortant de l'orifice de distribution 21 à des valeurs inférieures à 0,03 N, de préférence inférieures à 0,02 N.

Cette réduction de la force d'impaction s'accompagne d'une augmentation de la distribution des tailles des gouttelettes (DSD: Droplet Size Distribution), qui toutefois reste dans des valeurs acceptables, comprises entre 50 µm et 100 µm, de préférence entre 60 µm et 90 µm.

Le tableau 1 ci-dessous illustre les résultats de tests comparatifs entre un dispositif standard et deux dispositifs 1 et 2 selon la présente invention.

Le dispositif standard est tel que représenté sur la figure 1, avec un orifice de distribution comportant une hauteur h de 0,72 mm, de forme conique en se rétrécissant vers son extrémité axiale aval, et avec un diamètre minimal en sortie, au niveau de l'extrémité axiale aval, de 0,27 mm.

Les dispositifs 1 et 2 sont identiques au dispositif standard, sauf en ce qui concerne la géométrie de l'orifice de distribution 21.

Pour le dispositif 1, l'orifice de distribution 21 comporte un cylindre axial ayant une hauteur h de 0,6 mm et un diamètre d constant sur ladite hauteur h de 0,5 mm.

Pour le dispositif 2, l'orifice de distribution 21 comporte un cylindre axial ayant une hauteur h de 0,6 mm et un diamètre d constant sur ladite hauteur h de 0,4 mm.

La méthode de test a consisté à remplir le réservoir du dispositif de distribution avec de l'eau, puis à actionner le dispositif à l'aide d'une machine d'actionnement automatique à une vitesse d'actionnement réaliste pour l'utilisateur. Le spray d'eau émis a heurté une plaque située à 3 cm de distance de l'orifice de distribution du dispositif, ladite plaque étant associée à un capteur adapté à mesurer la force d'impaction. La dose d'eau émise sous forme de spray était de 50 µL, et la vitesse d'actionnement de la machine était de 70 mm/s.

Ce test comparatif a non seulement mesuré la force d'impaction, mais également la distribution des tailles des gouttelettes (DSD).

**Tableau 1**

| | **Hauteur h (mm)** | **Diamètre d (mm)** | **Vitesse d'actionnement (mm/s)** | **Volume dose (µL)** | **Force d'impaction (N)** | **DSD (µm)** |
|---|---|---|---|---|---|---|
| **Dispositif standard** | 0.72 | 0.27 | 70 | 50 | 0.066 | 30 |
| **Dispositif 1** | 0.60 | 0.50 | 70 | 50 | 0.019 | 90 |
| **Dispositif 2** | 0.60 | 0.40 | 70 | 50 | 0.026 | 60 |

Les résultats du test comparatif permettent d'établir qu'à dose constante, et à vitesse d'actionnement constante, la géométrie de l'orifice de distribution 21 a un impact important sur la force d'impaction du spray.

On constate ainsi que le dispositif 1 présente le meilleur résultat concernant la force d'impaction, à savoir inférieure à 0,02 N, notamment égale à 0,019 N, avec une DSD comprise entre 80 µm et 100 µm, notamment égale à 90 µm.

Le dispositif 2 a généré une force d'impaction légèrement supérieure, inférieure à 0,03 N, notamment égale à 0,026 N, mais toujours largement inférieure à celle du dispositif standard, à savoir 0,066 N, et avec une DSD comprise entre 50 µm et 70 µm, notamment égale à 60 µm.

La présente invention fournit donc un dispositif permettant de réduire sensiblement la force d'impaction du spray dans la narine, ce qui le rend acceptable pour une utilisation pédiatrique, notamment avec des nouveau-nés et des enfants en bas-âge.

Bien entendu, des variantes de réalisation sont envisageables, sans sortir du cadre de la présente invention, tel que défini par les revendications annexées.

## Revendications

1. Dispositif de distribution nasale de produit fluide, comportant:
- un réservoir (10) contenant une dose de produit fluide,
- une tête de distribution nasale (20) pourvue d'un orifice de distribution (21) comportant un cylindre axial (210) ayant une hauteur axiale (h) et un diamètre radial (d), et
- des moyens d'actionnement (30) prévus pour permettre l'actionnement du dispositif, lesdits moyens d'actionnement comportant un corps d'actionnement (31) mobile par rapport à la tête de distribution nasale, ledit corps d'actionnement (31) coopérant lors de l'actionnement avec ledit réservoir (10) pour le déplacer axialement par rapport à la tête de distribution nasale en direction de l'orifice de distribution,
la hauteur axiale (h) étant égale à 0,6 mm, le volume de dose étant de 50 µL et le diamètre radial (d) étant égal à 0,4 mm, et, pour une vitesse d'actionnement de 70 mm/s, la force d'impaction du spray mesurée à une distance de 3 cm dudit orifice de distribution (21) étant inférieure à 0,03 N, notamment égale à 0,026 N, et la distribution des tailles des gouttelettes étant comprise entre 50 µm et 70 µm, notamment égale à 60 µm.

2. Dispositif de distribution nasale de produit fluide, comportant:
- un réservoir (10) contenant une dose de produit fluide,
- une tête de distribution nasale (20) pourvue d'un orifice de distribution (21) comportant un cylindre axial (210) ayant une hauteur axiale (h) et un diamètre radial (d), et
- des moyens d'actionnement (30) prévus pour permettre l'actionnement du dispositif, lesdits moyens d'actionnement comportant un corps d'actionnement (31) mobile par rapport à la tête de distribution nasale, ledit corps d'actionnement (31) coopérant lors de l'actionnement avec ledit réservoir (10) pour le déplacer axialement par rapport à la tête de distribution nasale en direction de l'orifice de distribution,
la hauteur axiale (h) étant égale à 0,6 mm, le volume de dose étant de 50 pL et le diamètre radial (d) étant égal à 0,5 mm, et, pour une vitesse d'actionnement de 70 mm/s, la force d'impaction du spray mesurée à une distance de 3 cm dudit orifice de distribution (21) étant inférieure à 0,02 N, notamment égale à 0,019 N, et la distribution des tailles des gouttelettes étant comprise entre 80 µm et 100 µm, notamment égale à 90 µm.

3. Dispositif selon la revendication 1 ou 2, dans lequel ledit réservoir comporte un bouchon-piston (50) pour distribuer au moins une partie dudit produit fluide, ledit réservoir (10) étant, avant actionnement, obturé de manière étanche par ledit bouchon-piston (50).

4. Dispositif selon la revendication 3, comportant un insert (60) creux inséré et fixé dans ladite tête de distribution (20), ledit insert (60) supportant une canule (40) pour percer ledit bouchon-piston (50) et ainsi relier ledit réservoir (10) audit orifice de distribution (21) lors de l'actionnement.

## Patentansprüche

1. Vorrichtung zur nasalen Verabreichung eines flüssigen Produkts, umfassend:
- einen Vorratsbehälter (10), in dem eine Dosis des flüssigen Produkts enthalten ist,
- einen Nasalabgabekopf (20) mit einer Abgabeöffnung (21), die einen axialen Zylinder (210) mit einer axialen Höhe (h) und einem radialen Durchmesser (d) umfasst, und
- Betätigungsmittel (30), die zur Ermöglichung der Betätigung der Vorrichtung vorgesehen sind, wobei die Betätigungsmittel einen Betätigungskörper (31) umfassen, der in Bezug auf den Nasalabgabekopf beweglich ist, wobei bei der Betätigung der Betätigungskörper (31) mit dem Vorratsbehälter (10) zusammenwirkt, um diesen in Bezug auf den Nasalabgabekopf axial in Richtung zur Abgabeöffnung hin zu bewegen,
wobei die axiale Höhe (h) gleich 0,6 mm ist, das Dosisvolumen 50 µl beträgt und der radiale Durchmesser (d) gleich 0,4 mm ist, und
bei einer Betätigungsgeschwindigkeit von 70 mm/s die Aufprallkraft des Sprays, gemessen in einem Abstand von 3 cm von der Abgabeöffnung (21), unter 0,03 N liegt, insbesondere gleich 0,026 N ist, und die Verteilung der Tröpfchengrößen im Bereich von 50 µm bis 70 µm liegt, insbesondere gleich 60 µm beträgt.

2. Vorrichtung zur nasalen Verabreichung eines flüssigen Produkts, umfassend:
- einen Vorratsbehälter (10), in dem eine Dosis des flüssigen Produkts enthalten ist,
- einen Nasalabgabekopf (20) mit einer Abgabeöffnung (21), die einen axialen Zylinder (210) mit einer axialen Höhe (h) und einem radialen Durchmesser (d) aufweist, und
- Betätigungsmittel (30), die zur Ermöglichung der Betätigung der Vorrichtung vorgesehen sind, wobei die Betätigungsmittel einen Betätigungskörper (31) umfassen, der in Bezug auf den Nasalabgabekopf beweglich ist, wobei bei der Betätigung der Betätigungskörper (31) mit dem Vorratsbehälter (10) zusammenwirkt, um diesen in Bezug auf den Nasalabgabekopf axial in Richtung zur Abgabeöffnung hin zu bewegen,
wobei die axiale Höhe (h) gleich 0,6 mm ist, das Dosisvolumen 50 µl beträgt und der radiale Durchmesser (d) gleich 0,5 mm ist, und
bei einer Betätigungsgeschwindigkeit von 70 mm/s die Aufprallkraft des Sprays, gemessen in einem Abstand von 3 cm von der Abgabeöffnung (21), unter 0,02 N liegt, insbesondere gleich 0,019 N ist, und die Verteilung der Tröpfchengrößen im Bereich von 80 µm bis 100 µm liegt, insbesondere gleich 90 µm beträgt.

3. Vorrichtung nach Anspruch 1 oder 2, bei der der Vorratsbehälter einen Kolbenstopfen (50) zur Abgabe mindestens eines Teils des flüssigen Produkts umfasst, wobei der Vorratsbehälter (10) vor der Betätigung durch den Kolbenstopfen (50) dicht verschlossen ist.

4. Vorrichtung nach Anspruch 3, mit einem hohlen Einsatz (60), der in den Abgabekopf (20) eingesetzt und darin befestigt ist, wobei der Einsatz (60) eine Kanüle (40) trägt, die zum Durchstechen des Kolbenstopfens (50) dient, so dass der Vorratsbehälter (10) dann bei der Betätigung mit der Abgabeöffnung (21) verbunden ist.

## Claims

1. Device for nasal delivery of fluid product, comprising:
- a reservoir (10) containing a dose of fluid product,
- a nasal delivery head (20) provided with a delivery orifice (21) comprises an axial cylinder (210) having an axial height (h) and a radial diameter (d), and
- actuator means (30) to make it possible to actuate the device, said actuator means comprising an actuator body (31 that is movable relative to the nasal delivery head, said actuator body (31) engaging during actuation with said reservoir (10) so as to move it axially relative to the nasal delivery head towards the delivery orifice,
the axial height (h) being equal to 0.6mm, the dose volume being 50µL and the radial diameter (d) being equal to 0.4mm, and, for an actuation speed of 70mm/s, the force of the impact of the spray measured at a distance of 3cm from said delivery orifice (21) being less than 0.03N, in particular equal to 0.026N, and the distribution of the droplet sizes being of between 50µm and 70µm, in particular equal to 60µm.

2. Device for nasal delivery of fluid product, comprising:
- a reservoir (10) containing a dose of fluid product,
- a nasal delivery head (20) provided with a delivery orifice (21) comprises an axial cylinder (210) having an axial height (h) and a radial diameter (d), and
- actuator means (30) to make it possible to actuate the device, said actuator means comprising an actuator body (31 that is movable relative to the nasal delivery head, said actuator body (31) engaging during actuation with said reservoir (10) so as to move it axially relative to the nasal delivery head towards the delivery orifice,
the axial height (h) being equal to 0.6mm, the dose volume being 50µL and the radial diameter (d) being equal to 0.5mm, and, for an actuation speed of 70mm/s, the force of the impact of the spray measured at a distance of 3cm from said delivery orifice (21) being less than 0.02N, in particular equal to 0.019N, and the distribution of the droplet sizes being of between 80µm and 100µm, in particular equal to 90µm.

3. Device according to claim 1 or 2, wherein said reservoir comprises a stopper/piston (50) to deliver at least some of said fluid product, said reservoir (10) being, before actuation, sealingly blocked by said stopper/piston (50).

4. Device according to claim 3, comprising a hollow insert (60) inserted and fixed in said delivery head (20), said insert (60) supporting a cannula (40) to pierce said stopper/piston (50) and thus connect said reservoir (10) to said delivery orifice (21) during actuation.
